# EUROPEAN PATENT APPLICATION

(11) **EP 4 477 070 A1**
(43) Date of publication of application: **18.12.2024**
(21) Application number: 24176923.1
(22) Date of filing: 21.05.2024
(51) Int. Cl.: A01H 4/00, C12N 5/04

(54) **METHOD FOR PRODUCING MULTIPLE SHOOT CLUSTER AND METHOD FOR PRODUCING CLONAL SAPLING**

(30) Priority: 15.06.2023 JP 2023098468; 17.01.2024 JP 2024005227
(71) Applicant: Sumitomo Rubber Industries, Ltd., Kobe-shi, Hyogo 651-0072 (JP)
(72) Inventor: OKADA, Akari, Kobe-shi, 651-0072 (JP); TONO, Kaori, Kobe-shi, 651-0072 (JP)
(74) Representative: Manitz Finsterwald Patent- und Rechtsanwaltspartnerschaft mbB

(57) **Abstract**

Provided is a method for producing a multiple shoot cluster. A method for producing a multiple shoot cluster includes a shake culture step including shake-culturing a 1.0 cm to 5.0 cm tissue taken from *Hevea brasiliensis,* the tissue including at least one bud selected from the group consisting of apical buds, axillary buds, and dormant buds, in a MB basal medium or modified medium thereof containing a cytokinin plant hormone as a liquid medium.

## Description

### TECHNICAL FIELD

The present invention relates to a method for producing a multiple shoot cluster and a method for producing a clonal sapling.

### BACKGROUND ART

At present, natural rubber (a type of polyisoprenoid) used in industrial rubber products is obtained by cultivating rubber-producing plants, such as Para rubber trees (*Hevea brasiliensis*) of the family *Euphorbiaceae* or Indian rubber trees (*Ficus elastica*) among *Moraceae* plants, so that the laticifer cells of these plants biosynthesize natural rubber, and manually harvesting the natural rubber from the plants.

Natural rubber for industrial applications is currently sourced almost entirely from *Hevea brasiliensis.* Moreover, it is used widely and in large amounts in a variety of applications as the main raw material for rubber products. However, *Hevea brasiliensis* is a plant that can be grown only in limited regions such as Southeast Asia and South America. Furthermore, *Hevea brasiliensis* requires about seven years from planting to becoming mature enough for rubber extraction, and the collection season may be limited. The time period during which natural rubber can be collected from the mature tree is also limited to 20 to 30 years.

In the future, an increase in the demand for natural rubber mainly in developing countries is expected, and the exhaustion of natural rubber resources is a matter of concern. Thus, a stable source of supply for natural rubber is desired.

Under these circumstances, efforts have been made to increase the production of natural rubber by *Hevea brasiliensis.* For *Hevea brasiliensis,* sapling propagation is performed by raising and growing seedlings provided by seeding to yield a rootstock and grafting buds obtained from a clonal sapling to the rootstock.

Moreover, the grafted buds obtained from clonal saplings according to conventional clonal propagation techniques may simultaneously inherit diseases from the original trees, and diseased saplings may be propagated.

Furthermore, grafts cannot be true clonal saplings as they may be affected by rootstocks.

Meanwhile, micropropagation is known as a clonal sapling propagation technique using tissue culture. For example, in Patent Literature 1, a tissue containing a node, axillary bud, or apical bud of a rubber tree is cultured in an induction medium containing a plant growth hormone and a carbon source to form a shoot, which is then used to acquire a clonal sapling of the rubber tree. The use of the shoot can also produce a true clonal sapling.

### CITATION LIST

### PATENT LITERATURE

Patent Literature 1: JP 2016-140318 A

### SUMMARY OF INVENTION

### TECHNICAL PROBLEM

As a result of extensive studies on the method described in Patent Literature 1 in which a tissue containing a node, axillary bud, or apical bud of a rubber tree is cultured in an induction medium containing a plant growth hormone and a carbon source to form a shoot, the present inventors newly found that this method only induces a single shoot from a single tissue and cannot produce a multiple shoot cluster in which multiple shoots are formed.

The present invention aims to solve the problem newly found by the present inventors and provide a method for producing a multiple shoot cluster.

### SOLUTION TO PROBLEM

As a result of extensive studies, the present inventors have found that a multiple shoot cluster can be produced by shake-culturing a 1.0 cm to 5.0 cm tissue taken from *Hevea brasiliensis,* which has at least one bud selected from the group consisting of apical buds, axillary buds, and dormant buds, in a MB basal medium or modified medium thereof containing a cytokinin plant hormone as a liquid medium. This finding has led to the completion of the present invention.

That is, the present invention relates to a method for producing a multiple shoot cluster, which includes a shake culture step including shake-culturing a 1.0 cm to 5.0 cm tissue taken from *Hevea brasiliensis,* which has at least one bud selected from the group consisting of apical buds, axillary buds, and dormant buds, in a MB basal medium or modified medium thereof containing a cytokinin plant hormone as a liquid medium.

### ADVANTAGEOUS EFFECTS OF INVENTION

The method for producing a multiple shoot cluster of the present invention includes a shake culture step including shake-culturing a 1.0 cm to 5.0 cm tissue taken from *Hevea brasiliensis,* which has at least one bud selected from the group consisting of apical buds, axillary buds, and dormant buds, in a MB basal medium or modified medium thereof containing a cytokinin plant hormone as a liquid medium. Such a method can produce a multiple shoot cluster.

### BRIEF DESCRIPTION OF DRAWINGS

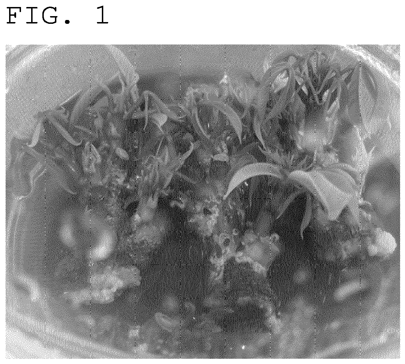

FIG. 1 is a photograph showing an exemplary multiple shoot cluster.

### DESCRIPTION OF EMBODIMENTS

The method for producing a multiple shoot cluster of the present invention includes a shake culture step including shake-culturing a 1.0 cm to 5.0 cm tissue taken from *Hevea brasiliensis,* which has at least one bud selected from the group consisting of apical buds, axillary buds, and dormant buds, in a MB basal medium or modified medium thereof containing a cytokinin plant hormone as a liquid medium. This method can produce from a single tissue a multiple shoot cluster in which multiple (e.g., two to six) shoots are formed. By individually cutting the shoots from the multiple shoot cluster, multiple shoots can be obtained from the single tissue. The multiple shoots can be used to produce clonal saplings of *Hevea brasiliensis.* This allows *Hevea brasiliensis* to be mass-propagated efficiently.

It should be noted that the production method of the present invention may include other steps as long as it includes the step specified above. The step specified above may be performed once or multiple times by subculture or other process.

The reason why the present invention provides the above-described advantageous effect is not clear, but is believed to be as follows.

When *Hevea brasiliensis,* which has a relatively strong apical dominance, is subjected to solid culture, only a single shoot can be induced from a single tissue, and therefore a multiple shoot cluster in which multiple shoots are formed cannot be produced. In solid culture, it is thus difficult to simultaneously activate multiple shoot primordia (e.g., apical bud and axillary bud) present in a tissue. As only a single shoot primordium (apical bud) can be activated, a single shoot can be induced, and it is difficult to increase the number of shoots and produce a multiple shoot cluster. Here, even if a tissue of *Hevea brasiliensis* not having an apical bud but having multiple axillary buds is subjected to solid culture, similarly, only a single shoot can be induced from a single tissue, and a multiple shoot cluster in which multiple shoots are formed cannot be produced. As described above, when a tissue of *Hevea brasiliensis* is subjected to solid culture, only a single shoot can be induced from a single tissue, and a multiple shoot cluster in which multiple shoots are formed cannot be produced.

Meanwhile, a method known to be effective for producing a multiple shoot cluster from a tissue is shoot tip culture, which involves cutting out a stem tip portion where cell division is active, and culturing this tissue. Although such shoot tip culture was applied to *Hevea brasiliensis,* a multiple shoot cluster could not be produced. The present inventors extensively investigated the reason for this and found that since *Hevea brasiliensis* is a plant that produces large quantities of latex, a shoot tip is affected by the latex secreted therefrom during the culture of the shoot tip. Thus, a multiple shoot cluster cannot be produced as a shoot tip is affected by the latex therefrom during the culture of the shoot tip. This problem is unique to *Hevea brasiliensis* and was newly found by the present inventors.

In order to solve the problem unique to *Hevea brasiliensis* newly found by the present inventors, the present inventors conducted extensive studies and found the following points.
(1) When a tissue taken from *Hevea brasiliensis,* which has at least one bud selected from the group consisting of apical buds, axillary buds, and dormant buds, is shake-cultured in a liquid medium, the tissue is soaked in the liquid medium, which allows the tissue to efficiently absorb nutrients from the medium, so that multiple shoot primordia (e.g., apical bud, axillary bud, dormant bud) present in the tissue of *Hevea brasiliensis* can be simultaneously activated, and the development of multiple shoots can be activated. This is presumably because shaking the tissue in the culture solution allows activation to be distributed among multiple buds, without being concentrated in one bud, even in the tissue of *Hevea brasiliensis* having a relatively strong apical dominance, thus making it possible to develop multiple shoots and produce a multiple shoot cluster in which multiple shoots are formed.
(2) When a tissue of *Hevea brasiliensis,* which is a plant that produces large quantities of latex, is used, the tissue needs to have an appropriate size for culture in order to fully achieve the effect described in (1). This matter is unique to *Hevea brasiliensis.* The size of the tissue of *Hevea brasiliensis* used for liquid culture needs to be large enough not to be easily affected by the latex, rather than being small like the shoot tip. On the other hand, if the size of the tissue of *Hevea brasiliensis* is too large, the sterilization efficiency may decrease during the sterilization step, which is important in tissue culture, leading to an increase in the risk of contamination. Also, if the size of the tissue of *Hevea brasiliensis* is too large, the tissue may not be shaken well in a liquid medium, leading to a reduction in oxygen supply to the tissue and inefficiency in nutrient supply. Therefore, presumably, the culture efficiency may decrease and the effect described in (1) cannot be fully achieved.
(3) Presumably, the effects described in (1) and (2) can be fully achieved by using a MB basal medium or modified medium thereof containing a cytokinin plant hormone as a liquid medium.

The synergistic effect of (1) to (3) presumably enables the production of a multiple shoot cluster in which multiple (e.g., two to six) shoots are formed from a single tissue of *Hevea brasiliensis.*

Moreover, in the shake culture step, in which the tissue of *Hevea brasiliensis* is shake-cultured in a liquid medium, multiple tissues can be simultaneously cultured in a single liquid medium. This also enables the efficient production of a multiple shoot cluster.

When the shoots of the multiple shoot cluster obtained by the production method of the present invention are elongated, the shoots tend to be glossy and have good growth conditions and a high rooting rate. In contrast, shoots conventionally produced using solid media tend to turn yellow easily.

The method for producing a multiple shoot cluster of the present invention includes a shake culture step including shake-culturing a 1.0 cm to 5.0 cm tissue taken from *Hevea brasiliensis,* which has at least one bud selected from the group consisting of apical buds, axillary buds, and dormant buds, in a MB basal medium or modified medium thereof containing a cytokinin plant hormone as a liquid medium.

Herein, the term "shoot" refers to an elongated bud. Herein, the term "multiple shoot cluster" refers to a tissue in which multiple shoots are formed.

The tissue used in the present invention is a tissue taken from *Hevea brasiliensis* and has at least one bud selected from the group consisting of apical buds, axillary buds, and dormant buds. The size of the tissue is 1.0 cm to 5.0 cm. This means that, when the tissue includes at least one bud selected from the group consisting of apical buds, axillary buds, and dormant buds, the tissue necessarily has multiple shoot primordia (e.g., buds).

The tissue preferably has at least one bud selected from the group consisting of apical buds and axillary buds, and more preferably has an apical bud.

The number of buds in the tissue is preferably two or more, more preferably three or more, still more preferably four or more, particularly preferably six or more. The upper limit of the number of buds is not limited because as the number of buds increases, the number of shoots is increased. Here, the tissue may have one type of bud or multiple types of buds. In other words, the tissue may have only axillary buds, or may have apical and axillary buds.

The size of the tissue is 1.0 cm to 5.0 cm. The lower limit of the size of the tissue is preferably 1.5 cm or more, more preferably 2.0 cm or more. The upper limit thereof is preferably 4.5 cm or less, more preferably 3.5 cm or less.

Herein, the size of the tissue refers to the number average value of multiple tissues determined by viewing each tissue in plan view from various angles; measuring the length of the longest of the straight lines each connecting any two points located on the outer edges of the tissue on each plan view; and dividing the sum of the measured lengths by the number of tissues measured.

Specific examples of the tissue include tissues derived from mature trees, young trees, saplings, or clonal saplings of *Hevea brasiliensis,* and tissues derived from aseptic in vitro plantlets grown in vitro from seedlings of *Hevea brasiliensis.*

When the tissues derived from mature trees, young trees, saplings, or clonal saplings are used, they may be cut to the size described above, followed by disinfection or sterilization of the surface before use. When the tissues derived from aseptic in vitro plantlets grown in vitro from seedlings are used, they may be cut to the size described above before use.

When the tissues derived from mature trees, young trees, saplings, or clonal saplings are used, the tissue surface is first cleaned before culture in a below-described liquid medium. For example, the surface may be cleaned with an abrasive powder or a soft sponge, but is preferably cleaned with running water. The water used for cleaning may contain approximately 0.1% by mass of surfactant(s).

Next, the tissue is disinfected or sterilized. The disinfection or sterilization may be performed using known disinfectants or sterilizing agents, preferably ethanol, benzalkonium chloride, or an aqueous sodium hypochlorite solution. Here, the disinfection or sterilization treatment may further be followed by washing with sterile water.

As a specific example, the cleaning and disinfection or sterilization treatment may be performed by the following procedure: clean the tissue surface with running water and wash the tissue with ethanol; then sterilize the tissue using an aqueous sodium hypochlorite solution optionally with stirring; and thereafter wash the tissue with sterile water.

### (Shake culture step (induction step))

In the shake culture step, the tissue is shake-cultured in a MB basal medium or modified medium thereof containing a cytokinin plant hormone as a liquid medium to induce and form multiple shoots, thereby producing a multiple shoot cluster. Here, when a disinfected or sterilized tissue is used, the cut end is preferably cut off to eliminate the effect of the disinfectant or sterilizing agent before culture.

A liquid medium is used in the shake culture step. The liquid medium is not limited as long as it is a MB basal medium (as described in Biotechnology in Agriculture and Forestry volume 5 (Trees II), pp. 222-245) or modified medium thereof containing a cytokinin plant hormone. Hereinafter, the MB basal medium and the modified medium of the MB basal medium are also collectively referred to as the base medium.

Herein, the term "modified medium of the MB basal medium" refers to a medium obtained by altering the composition of the MB basal medium, specifically a medium containing the components contained in the MB basal medium. More specifically, it refers to a medium containing the components contained in the MB basal medium, with the amount of each component falling within 0.1 to 10, preferably 0.4 to 5.0, more preferably 0.5 to 2.0, relative to the amount of the corresponding component in the basal medium, which is taken as 1. The same applies to modified media of other media.

The liquid medium used may be prepared by adding a cytokinin plant hormone, which is a plant growth hormone, to the base medium.

Examples of the cytokinin plant hormone include benzyladenine, kinetin, zeatin, benzylaminopurine, isopentenylaminopurine, thidiazuron, isopentenyladenine, zeatin riboside, and dihydrozeatin. These may be used alone or in combinations of two or more. Among these, benzyladenine, kinetin, and zeatin are preferred, benzyladenine and kinetin are more preferred, and benzyladenine is still more preferred.

The cytokinin plant hormone concentration in the liquid medium is preferably at least 0.01 mg/L, more preferably at least 0.1 mg/L, still more preferably at least 0.5 mg/L, particularly preferably at least 0.8 mg/L, most preferably at least 3.0 mg/L. The cytokinin plant hormone concentration is preferably not more than 8.0 mg/L, more preferably not more than 7.0 mg/L, still more preferably not more than 6.0 mg/L.

In particular, when the cytokinin plant hormone used is benzyladenine, the benzyladenine concentration is preferably 4.0 to 6.0 mg/L, most preferably 5.0 mg/L. Moreover, when the cytokinin plant hormone used is kinetin, the kinetin concentration is preferably 0.8 to 1.2 mg/L, most preferably 1.0 mg/L.

The liquid medium may contain plant growth hormones other than cytokinin plant hormones. Examples of the plant growth hormones other than cytokinin plant hormones include auxin plant hormones.

Examples of the auxin plant hormones include 2,4-dichlorophenoxyacetic acid, 1-naphthaleneacetic acid, indole-3-butyric acid, indole-3-acetic acid, indolepropionic acid, chlorophenoxyacetic acid, naphthoxyacetic acid, phenylacetic acid, 2,4,5-trichlorophenoxyacetic acid, para-chlorophenoxyacetic acid, 2-methyl-4-chlorophenoxyacetic acid, 4-fluorophenoxyacetic acid, 2-methoxy-3,6-dichlorobenzoic acid, 2-phenyl acid, picloram, and picolinic acid. These may be used alone or in combinations of two or more. Among these, 2,4-dichlorophenoxyacetic acid, 1-naphthaleneacetic acid, and indole-3-butyric acid are preferred, and 2,4-dichlorophenoxyacetic acid or 1-naphthaleneacetic acid is more preferred.

Preferably, substantially no auxin plant hormone is added to the liquid medium. Specifically, the auxin plant hormone concentration in the liquid medium is preferably not more than 1.0 mg/L, more preferably not more than 0.1 mg/L, still more preferably not more than 0.05 mg/L, particularly preferably not more than 0.01 mg/L.

Preferably, the liquid medium contains a carbon source.

Any carbon source may be used, including sugars such as sucrose, glucose, trehalose, fructose, lactose, galactose, xylose, allose, talose, gulose, altrose, mannose, idose, arabinose, apiose, mannitol, sorbitol, xylitol, erythritol, and maltose. These may be used alone or in combination of two or more. Sucrose is preferred among these.

The carbon source (preferably sucrose) concentration in the liquid medium is preferably at least 0.1% by mass, more preferably at least 1.0% by mass, still more preferably at least 3.0% by mass. The carbon source concentration is preferably not more than 10% by mass, more preferably not more than 9.0% by mass, still more preferably not more than 5.0% by mass. Herein, the term "carbon source concentration" refers to the concentration of sugars.

Preferably, the liquid medium contains at least one selected from the group consisting of amino acids and vitamins. These may be used alone or in combination of two or more.

The amino acids are not limited as long as they are organic compounds having both an amino group and a carboxy group as functional groups. Examples include aspartic acid, glutamine, glutamic acid, asparagine, valine, leucine, isoleucine, arginine, lysine, and phenylalanine. These may be used alone or in combination of two or more. Glutamine is preferred among these.

Here, although the amino acids may be L- or D-amino acids, L-amino acids are preferred because they are abundant in nature and are easy to use as a biomass resource.

The amino acid concentration in the liquid medium is preferably at least 20 mg/L, more preferably at least 80 mg/L, still more preferably at least 100 mg/L. The amino acid concentration is preferably not more than 1000 mg/L, more preferably not more than 800 mg/L, still more preferably not more than 400 mg/L.

Non-limiting examples of the vitamins include biotin, thiamine (vitamin B1), pyridoxine (vitamin B4), pyridoxal, pyridoxamine, calcium pantothenate, inositol, nicotinic acid, nicotinic acid amide, and riboflavin (vitamin B2). These may be used alone or in combination of two or more. Thiamine is preferred among these.

The vitamin concentration in the liquid medium is preferably at least 1 mg/L, more preferably at least 4 mg/L, still more preferably at least 8 mg/L. The vitamin concentration is preferably not more than 100 mg/L, more preferably not more than 80 mg/L, still more preferably not more than 40 mg/L.

The liquid medium may further contain activated carbon in order to prevent growth inhibitors from accumulating in the tissue. Moreover, the liquid medium preferably further contains silver nitrate in order to promote shoot formation. Furthermore, the liquid medium may contain coconut water (coconut milk) in order to promote shoot formation.

The silver nitrate concentration in the liquid medium is preferably at least 0.1 mg/L, more preferably at least 0.3 mg/L, still more preferably at least 0.5 mg/L. The silver nitrate concentration is preferably not more than 5.0 mg/L, more preferably not more than 3.0 mg/L.

The pH of the liquid medium is preferably 4.0 to 10.0, more preferably 5.0 to 6.5, still more preferably 5.5 to 6.0.

In the shake culture step, the tissue is shake-cultured in the liquid medium. The shaking speed in the shake culture is preferably 50 rpm or higher, more preferably 65 rpm or higher, still more preferably 80 rpm or higher, while it is preferably 200 rpm or lower, more preferably 170 rpm or lower, still more preferably 140 rpm or lower.

The shake culture step is usually performed in a controlled environment in which culture conditions such as temperature and light cycle are managed. The culture conditions may be selected as appropriate, but, for example, the culture temperature is preferably 0°C to 40°C, more preferably 20°C to 40°C, still more preferably 25°C to 35°C. Culture may be performed in the dark or light, but, for example, the light conditions may include a light cycle of 14 to 16 hours light at 12.5 µmol/m²/s. The culture duration is not limited, but culture for one to eight weeks is preferred, and culture for two to four weeks is more preferred.

Among the conditions indicated above, it is particularly preferred that the plant growth hormone is benzyladenine at a concentration of 3.0 mg/L to 8.0 mg/L and the culture temperature is 25°C to 35°C.

As described above, shake-culturing the tissue in the liquid medium enables the induction and formation of multiple shoots from a single tissue and thus the production of a multiple shoot cluster in which multiple (e.g., two to six) shoots are formed from a single tissue.

### (Shoot culture step)

In the present invention, a shoot culture step may optionally be performed in which the multiple shoot cluster obtained in the shake culture step is cultured in a shoot culture medium. This enables the elongation of the shoots of the multiple shoot cluster.

In the shoot culture step, the multiple shoot cluster formed through the shake culture step is cultured in a shoot culture medium, preferably a shoot culture medium containing a plant growth hormone and a carbon source. Specifically, the multiple shoot cluster formed through the shake culture step can be inserted, transplanted, and cultured in the shoot culture medium to elongate the shoots of the multiple shoot cluster and also to acquire new buds. Then, once the shoots of the multiple shoot cluster are elongated, the elongated shoots may be cut from the multiple shoot cluster and individually subjected to the shoot culture step. This allows each shoot to further elongate. In this way, multiple shoots can be obtained from a single tissue of *Hevea brasiliensis.* These multiple shoots can be rooted to produce clonal saplings of *Hevea brasiliensis,* thus enabling efficient mass propagation of *Hevea brasiliensis.*

Of course, the shake culture step may also be continued to elongate the shoots of the multiple shoot cluster. The elongated shoots can be cut from the multiple shoot cluster and rooted to produce clonal saplings of *Hevea brasiliensis,* thus enabling efficient mass propagation of *Hevea brasiliensis.* Here, of course, the cut shoots may be individually subjected to the shoot culture step before rooting.

The shoot culture step may be performed according to a known method, for example, the method described in JP 2020-036545 A.

The shoot culture medium may be liquid or solid. Solid culture is preferred because elongation of the shoots is facilitated when the shoots are inserted and cultured in the medium. Moreover, when the shoot culture medium is a liquid medium, static culture or shake culture may be performed.

Preferably, the shoot culture medium contains a plant growth hormone and a carbon source. Examples of the plant growth hormone include auxin plant hormones and/or cytokinin plant hormones. Cytokinin plant hormones are preferred among these.

The auxin plant hormones may be as described for the auxin plant hormones usable in the liquid medium.

The cytokinin plant hormones may be as described for the cytokinin plant hormones usable in the liquid medium. In particular, benzyladenine, kinetin, and zeatin are preferred, benzyladenine and kinetin are more preferred, and benzyladenine is still more preferred.

Any carbon source may be used in the shoot culture medium, including those as described for the carbon sources usable in the liquid medium. Sucrose is preferred among these.

Like the liquid medium, the shoot culture medium preferably further contains activated carbon and/or silver nitrate.

Examples of the shoot culture medium include those prepared by adding plant growth hormones to base media, including: basal media such as White's medium (disclosed on pp. 20-36 of Shokubutsu Saibo Kogaku Nyumon (Introduction to Plant Cell Engineering), Japan Scientific Societies Press), Heller's medium (Heller R, Bot. Biol. Veg. Paris 14, 1-223 (1953)), SH medium (Schenk and Hildebrandt medium), MS medium (Murashige and Skoog medium) (disclosed on pp. 20-36 of Shokubutsu Saibo Kogaku Nyumon (Introduction to Plant Cell Engineering), Japan Scientific Societies Press), LS medium (Linsmaier and Skoog medium) (disclosed on pp. 20-36 of Shokubutsu Saibo Kogaku Nyumon (Introduction to Plant Cell Engineering), Japan Scientific Societies Press), Gamborg medium, B5 medium (disclosed on pp. 20-36 of Shokubutsu Saibo Kogaku Nyumon (Introduction to Plant Cell Engineering), Japan Scientific Societies Press), MB medium (disclosed on pp. 222-245 of Biotechnology in Agriculture and Forestry volume 5 (TreesII)), and WP medium (for woody plants); and modified basal media obtained by altering the composition of the basal media. Preferred among these are those prepared by adding plant growth hormones to MS medium, B5 medium, WP medium, or MB medium. More preferred are those prepared by adding plant growth hormones to MS medium, modified MS media obtained by altering the composition of the MS medium, MB medium, or modified MB media obtained by altering the composition of the MB medium. Still more preferred are those prepared by adding plant growth hormones to MB medium or modified MB media obtained by altering the composition of the MB medium.

When the shoot culture medium is used as a solid medium, the medium may be solidified using a solidifying agent. Any solidifying agent may be used, including Japanese gelatin, agar, gellan gum, agarose, Gelrite, agar, and Phytagel.

The following describes the common composition and culture conditions of a suitable shoot culture medium.

The carbon source (preferably sucrose) concentration in the shoot culture medium is preferably at least 3.0% by mass, more preferably at least 5.0% by mass. The carbon source (preferably sucrose) concentration is preferably not more than 9.0% by mass, more preferably not more than 7.0% by mass.

The auxin plant hormone concentration in the shoot culture medium is preferably not more than 2.0 mg/L, more preferably not more than 1.0 mg/L, still more preferably not more than 0.1 mg/L, particularly preferably not more than 0.08 mg/L, most preferably 0 mg/L.

When a cytokinin plant hormone is added to the shoot culture medium, the cytokinin plant hormone concentration in the shoot culture medium is preferably at least 1.0 mg/L, more preferably at least 3.0 mg/L, still more preferably at least 3.5 mg/L, particularly preferably at least 4.0 mg/L. The cytokinin plant hormone concentration is preferably not more than 10 mg/L, more preferably not more than 8.0 mg/L, still more preferably not more than 6.0 mg/L.

The activated carbon concentration in the shoot culture medium is preferably at least 0.01% by mass, more preferably at least 0.03% by mass. The activated carbon concentration is preferably not more than 1.0% by mass, more preferably not more than 0.1% by mass.

The silver nitrate concentration in the shoot culture medium is preferably at least 0.1 mg/L, more preferably at least 0.3 mg/L, still more preferably at least 0.5 mg/L. The silver nitrate concentration is preferably not more than 5.0 mg/L, more preferably not more than 3.0 mg/L.

The pH of the shoot culture medium is preferably 4.0 to 10.0, more preferably 5.0 to 6.5, still more preferably 5.5 to 6.0.

The shoot culture step is usually performed in a controlled environment in which culture conditions such as temperature and light cycle are managed. The culture conditions may be selected as appropriate, but, for example, the culture temperature is preferably 0°C to 40°C, more preferably 20°C to 40°C, still more preferably 25°C to 35°C. Culture may be performed in the dark or light, but, for example, the light conditions may include a light cycle of 14 to 16 hours light at 5 to 20 µmol/m²/s.

When the shoot culture medium is a solid medium, the solidifying agent concentration in the shoot culture medium is preferably at least 0.1% by mass, more preferably at least 0.2% by mass. The solidifying agent concentration is preferably not more than 2.0% by mass, more preferably not more than 1.1% by mass, still more preferably not more than 0.8% by mass.

Among the conditions indicated above, it is preferred that the shoot culture medium is MB medium or a modified MB medium obtained by altering the composition of the MB medium, more preferably a medium prepared by adding a plant growth hormone to MB medium or a modified MB medium obtained by altering the composition of the MB medium, still more preferably a medium prepared by adding a cytokinin plant hormone to MB medium or a modified MB medium obtained by altering the composition of the MB medium.

As described above, the shoots can be elongated by the shoot culture step. The shoots elongated by the shoot culture step are shoots that have grown stably, and thus can be suitably subjected to a rooting step.

### (Rooting step)

In the rooting step, the shoots cut from the multiple shoot cluster are cultured in a rooting induction medium to cause rooting. Here, the shoots cut from the multiple shoot cluster may or may not have undergone the shoot culture step.

The rooting method is not limited. An exemplary rooting step is described.

In the rooting step, for example, the shoots cut from the multiple shoot cluster are cultured in a rooting induction medium to cause rooting. Here, the rooting induction medium may be liquid or solid. Solid culture is preferred because rooting is facilitated when the shoots are inserted and cultured in the medium. Moreover, when the rooting induction medium is a liquid medium, static culture or shake culture may be performed.

The rooting induction medium usually contains a plant growth hormone and a carbon source, and examples of the plant growth hormone include auxin plant hormones and/or cytokinin plant hormones. Among these, auxin plant hormones are preferred.

The auxin plant hormones may be as described for the auxin plant hormones usable in the liquid medium. In particular, 2,4-dichlorophenoxyacetic acid, 1-naphthaleneacetic acid, indole-3-butyric acid, and indole-3-acetic acid are preferred, and indole-3-butyric acid is more preferred.

The cytokinin plant hormones may be as described for the cytokinin plant hormones usable in the liquid medium. In particular, benzyladenine, kinetin, and zeatin are preferred, and benzyladenine or kinetin is more preferred.

Any carbon source may be used in the rooting induction medium, including those as described for the carbon sources usable in the liquid medium. Sucrose is preferred among these.

Like the liquid medium, the rooting induction medium preferably further contains activated carbon and/or silver nitrate.

The rooting induction medium preferably contains glutathione. This can lead to a better rooting ratio.

The glutathione is a tripeptide composed of glutamic acid, cysteine, and glycine as constituent amino acids and may be reduced glutathione, oxidized glutathione (glutathione disulfide), or a mixture thereof, preferably reduced glutathione.

Examples of the rooting induction medium include those prepared by adding plant growth hormones to base media such as the basal media usable as the shoot culture medium and modified basal media obtained by altering the composition of the basal media. Preferred among these are those prepared by adding plant growth hormones to MS medium, B5 medium, or WP medium. More preferred are those prepared by adding plant growth hormones to MS medium or modified MS media obtained by altering the composition of the MS medium. Also preferred are MB medium or modified MB media obtained by altering the composition of the MB medium. More preferred are those prepared by adding carbon sources and/or glutathione to MB medium or modified MB media obtained by altering the composition of the MB medium.

When the rooting induction medium is used as a solid medium, the medium may be solidified using a solidifying agent. Any solidifying agent may be used, including Japanese gelatin, agar, gellan gum, agarose, Gelrite, agar, and Phytagel.

The following describes the common composition and culture conditions of a suitable rooting induction medium.

The carbon source concentration in the rooting induction medium is preferably at least 0.1% by mass, more preferably at least 1.0% by mass. The carbon source concentration is preferably not more than 10% by mass, more preferably not more than 5.0% by mass.

When an auxin plant hormone is added to the rooting induction medium, the auxin plant hormone concentration in the rooting induction medium is preferably at least 0.5 mg/L, more preferably at least 1.0 mg/L, still more preferably at least 3.0 mg/L. The auxin plant hormone concentration is preferably not more than 10 mg/L, more preferably not more than 6.0 mg/L, still more preferably not more than 5.0 mg/L.

Preferably, substantially no cytokinin plant hormone is added to the rooting induction medium. Specifically, the cytokinin plant hormone concentration is preferably not more than 1.0 mg/L, more preferably not more than 0.1 mg/L, still more preferably not more than 0.05 mg/L, particularly preferably not more than 0.01 mg/L.

The activated carbon concentration in the rooting induction medium is preferably at least 0.01% by mass, more preferably at least 0.03% by mass. The activated carbon concentration is preferably not more than 1.0% by mass, more preferably not more than 0.1% by mass.

The silver nitrate concentration in the rooting induction medium is preferably at least 0.1 mg/L, more preferably at least 0.3 mg/L, still more preferably at least 0.5 mg/L. The silver nitrate concentration is preferably not more than 5.0 mg/L, more preferably not more than 3.0 mg/L.

The glutathione concentration in the rooting induction medium is preferably at least 10 µmol/L, more preferably at least 30 µmol/L, still more preferably at least 40 µmol/L, particularly preferably at least 60 µmol/L, most preferably at least 80 µmol/L, but is preferably not more than 500 µmol/L, more preferably not more than 400 µmol/L, still more preferably not more than 300 µmol/L, particularly preferably not more than 200 µmol/L, most preferably not more than 150 µmol/L. This can lead to a better rooting ratio.

The pH of the rooting induction medium is preferably 4.0 to 10.0, more preferably 5.0 to 6.5, still more preferably 5.5 to 6.0.

The rooting step is usually performed in a controlled environment in which culture conditions such as temperature and light cycle are managed. The culture conditions may be selected as appropriate, but, for example, the culture temperature is preferably 0°C to 40°C, more preferably 20°C to 40°C, still more preferably 25°C to 35°C. Culture may be performed in the dark or light, but, for example, the light conditions may include a light cycle of 14 to 16 hours light at 12.5 µmol/m²/s. The culture duration is not limited, but culture for one to ten weeks is preferred, and culture for four to eight weeks is more preferred.

The solidifying agent concentration in the rooting induction medium as a solid medium is preferably at least 0.1% by mass, more preferably at least 0.2% by mass, still more preferably at least 0.5% by mass. The solidifying agent concentration is preferably not more than 2.0% by mass, more preferably not more than 1.1% by mass, still more preferably not more than 0.8% by mass.

Among the conditions indicated above, it is particularly preferred that the plant growth hormone is an auxin plant hormone, particularly indole-3-butyric acid, at a concentration of 3.0 mg/L to 6.0 mg/L and the culture temperature is 25°C to 35°C.

As described above, the shoots can be cultured in the rooting induction medium to cause rooting, thereby producing rooted shoots (herein, the rooted shoots are also referred to as "plantlets"). Thus, clonal saplings, which are complete plants, can be formed. The plantlets may be directly transplanted into soil, or may be subjected to acclimatization before transplanting into soil. The acclimatization may be performed by any method.

Here, the clonal saplings thus formed can also be repeatedly subjected to the shake culture step and rooting step or to the shake culture step, shoot culture step, and rooting step to stably mass-produce clonal saplings of superior varieties.

As described above, the shoots can be cultured in the rooting induction medium to cause rooting. Preferably, the rooting step includes:
a rooting induction step including soaking the shoots cut from the produced multiple shoot cluster in a rooting induction solution containing an auxin plant hormone; and
a rooting induction culture step including culturing the shoots treated in the rooting induction step in a rooting induction medium. This can lead to more suitable shoot rooting and a better rooting ratio.

### <Rooting induction step>

In the rooting induction step, the shoots (shoot pieces) cut from the produced multiple shoot cluster are soaked in a rooting induction solution containing an auxin plant hormone. This enables more suitable promotion of shoot rooting.

The shoots are preferably soaked in the rooting induction solution so that the end of each shoot piece, namely, the cut end of each shoot, is submerged in the rooting induction solution although the shoots may be soaked so that the cut end of each shoot is not submerged in the rooting induction solution.

Moreover, the shoots may be soaked in the rooting induction solution while the shoots are allowed to stand still or while the shoots are shaken. To prevent the multiple shoots from getting entangled with each other and from damaging the growth point, the shoots are preferably soaked while they are allowed to stand still.

Preferably, the shoots are soaked in the rooting induction solution so that 30% to 70% of the volume of each shoot, taken as 100%, is submerged in the rooting induction solution. More preferably, the shoots are soaked so that the end of each shoot piece, namely, the cut end of each shoot, is submerged in the rooting induction solution, and 30% to 70% of the volume of each shoot, taken as 100%, is submerged in the rooting induction solution.

When 30% to 70% of the volume of each shoot, taken as 100%, is submerged in the rooting induction solution, it tends to be possible to increase the auxin activity of not only the basal part but also the entire shoot, thereby enabling more suitable promotion of shoot rooting. More preferably, at least 40% of the volume of each shoot, taken as 100%, is submerged in the rooting induction solution, and more preferably, not more than 60% of the volume of each shoot, taken as 100%, is submerged in the rooting induction solution.

The duration of the rooting induction step is preferably 12 hours or longer, more preferably 20 hours or longer, still more preferably 24 hours or longer, but is preferably 96 hours or shorter, more preferably 84 hours or shorter, still more preferably 72 hours or shorter. This can lead to a better rooting ratio.

The rooting induction step is preferably performed in a controlled environment in which conditions such as temperature and light cycle are managed. For example, the temperature in the rooting induction step is preferably 0°C to 40°C, more preferably 20°C to 40°C, still more preferably 25°C to 35°C. The rooting induction step may be performed in the dark or light, but, for example, the light conditions may include a light cycle of 14 to 16 hours light at 5 to 20 µmol/m²/s.

The length of the shoot (shoot piece) to be subjected to the rooting induction step is preferably 10 mm or longer, more preferably 15 mm or longer, still more preferably 20 mm or longer, but is preferably 100 mm or shorter, more preferably 80 mm or shorter, still more preferably 50 mm or shorter. This can lead to a better rooting ratio.

The auxin plant hormone may be as described for the auxin plant hormones usable in the liquid medium. In particular, indole-3-acetic acid is preferred. By using indole-3-acetic acid, it tends to be possible to increase the auxin activity of the entire shoot without weakening the entire shoot even during the rooting induction step, thereby enabling more suitable promotion of shoot rooting. It is also preferred to use 1-naphthaleneacetic acid or indole-3-butyric acid together with indole-3-acetic acid.

The auxin plant hormone concentration in the rooting induction solution is preferably 15 to 20 mg/L. Due to such a relatively high auxin plant hormone concentration in the rooting induction solution, it tends to be possible to increase the auxin activity of the entire shoot, thereby enabling more suitable promotion of shoot rooting. In particular, the concentration of auxin plant hormones including indole-3-acetic acid in the rooting induction solution is preferably 15 to 20 mg/L. This tends to enable more suitable promotion of shoot rooting.

Here, when multiple auxin plant hormones are used, the auxin plant hormone concentration means the total concentration of auxin plant hormones. The same applies to other similar descriptions.

The rooting induction solution preferably contains a cytokinin plant hormone. This tends to enable more suitable promotion of shoot rooting.

The cytokinin plant hormone may be as described for the cytokinin plant hormones usable in the liquid medium. In particular, zeatin is preferred.

When a cytokinin plant hormone is added to the rooting induction solution, the cytokinin plant hormone concentration in the rooting induction solution is preferably at least 0.01 mg/L, more preferably at least 0.05 mg/L. The cytokinin plant hormone concentration is preferably not more than 1 mg/L, more preferably not more than 0.5 mg/L, still more preferably not more than 0.2 mg/L.

The auxin plant hormone concentration in the rooting induction solution is preferably 15 to 20 mg/L. Preferably, the auxin plant hormone concentration satisfies the above numerical range and further satisfies any of the following (1) to (3), more preferably any of the following (2) and (3), still more preferably the following (3).
(1) The indole-3-acetic acid concentration in the rooting induction solution is 3 to 7 mg/L, the 1-naphthaleneacetic acid concentration in the rooting induction solution is 3 to 7 mg/L, and the indole-3-butyric acid concentration in the rooting induction solution is 3 to 7 mg/L.
(2) The indole-3-acetic acid concentration in the rooting induction solution is 15 to 20 mg/L.
(3) The indole-3-acetic acid concentration in the rooting induction solution is 15 to 20 mg/L and the zeatin concentration in the rooting induction solution is 0.01 to 1 mg/L.

The rooting induction solution may contain any other component, preferably glutathione. This can lead to a better rooting ratio.

The glutathione is a tripeptide composed of glutamic acid, cysteine, and glycine as constituent amino acids and may be reduced glutathione, oxidized glutathione (glutathione disulfide), or a mixture thereof, preferably reduced glutathione.

The glutathione concentration in the rooting induction solution is preferably at least 10 µmol/L, more preferably at least 30 µmol/L, still more preferably at least 40 µmol/L, particularly preferably at least 60 µmol/L, most preferably at least 80 µmol/L, but is preferably not more than 500 µmol/L, more preferably not more than 400 µmol/L, still more preferably not more than 300 µmol/L, particularly preferably not more than 200 µmol/L, most preferably not more than 150 µmol/L. This can lead to a better rooting ratio.

It is sufficient that the rooting induction solution contain an auxin plant hormone, and any dispersion medium may be used to dissolve the auxin plant hormone, including water, isotonic solutions, buffers, and tissue culture media. Examples of isotonic solutions include liquids prepared by adding an inorganic salt such as KCl, NaCl, CaCl₂, or MgCl₂ to a concentration of 0.01 to 7 M, preferably 0.5 to 2 M. Examples of buffers include phosphate buffer, Tris buffer, and MES buffer. Examples of tissue culture media include the above-mentioned media. To achieve the advantageous effect more suitably, tissue culture media are preferred. In other words, the rooting induction solution is preferably an aqueous solution in which an auxin plant hormone is dissolved in a tissue culture medium.

The tissue culture medium used may be as described for the shoot culture medium. In particular, it is preferably MS medium or a modified MS medium obtained by altering the composition of the MS medium.

### <Rooting induction culture step>

In the rooting induction culture step, the shoots treated in the rooting induction step are cultured in a rooting induction medium. This causes rooting of the shoots.

Here, although the rooting induction medium may be liquid or solid, solid culture is preferred because rooting is facilitated when the shoots are inserted and cultured in the medium. Moreover, when the rooting induction medium is a liquid medium, static culture or shake culture may be performed.

The rooting induction medium contains a carbon source and is basically as described above for the rooting induction medium. Also, the culture conditions in the rooting induction culture step are as described above for the rooting step.

In the present embodiment in which the rooting induction step is performed, the plant hormone concentration in the rooting induction medium is preferably not more than 2.0 mg/L, more preferably not more than 1.0 mg/L, still more preferably not more than 0.1 mg/L, particularly preferably not more than 0.08 mg/L, most preferably 0 mg/L. This can lead to a better rooting ratio.

Among the conditions indicated above, it is preferred that the plant hormone concentration is low (substantially no plant hormone is present).

The rooting induction medium may also be a solid medium containing a medium component. This tends to lead to a better shoot rooting ratio.

The solid medium may be any medium insoluble in water. Examples include known culture soils, and specific examples include culture soils prepared by appropriately mixing at least one selected from the group consisting of sand, peat moss, activated carbon, dolomite, isolite, bentonite, zeolite, perlite, vermiculite, ceramic, coconut fibers, bark media, rice husks, rock wool, and other various soil improvement materials, etc.

In addition to the above-mentioned culture soils, examples of usable solid media include resin media such as granular, foam, or sponge resins; and media prepared by solidifying liquid media (e.g., the above-described basal media) using solidifying agents.

These solid media may be used alone or in combinations of two or more. Moreover, the solid medium used may be uncompressed or may be compressed at an appropriate compression ratio.

Non-limiting examples of the resins include polyethylene resins, polypropylene resins, polystyrene resins, polyurethane resins, phenolic resins, and polyester resins.

Non-limiting examples of the solidifying agents include Japanese gelatin, agar, gellan gum, agarose, Gelrite, gelatin, and silica gel.

The solid medium is preferably a culture soil, more preferably vermiculite, peat moss, or rock wool, still more preferably vermiculite.

The wet density of the solid medium containing a medium component is preferably 0.4 g/cm³ or more, more preferably 0.5 g/cm³ or more, still more preferably 0.6 g/cm³ or more, but is preferably 1.0 g/cm³ or less, more preferably 0.9 g/cm³ or less, still more preferably 0.8 g/cm³ or less. Such a solid medium can moderately stimulate the roots and also contains oxygen, and therefore the advantageous effect tends to be achieved more suitably.

Herein, the wet density of the solid medium containing a medium component refers to the mass per unit volume of the entire solid medium. Specifically, it is measured by a caliper method in accordance with JIS A 1225 (2020).

The average particle size of the solid medium containing a medium component is preferably 0.25 mm or more, more preferably 0.5 mm or more, still more preferably 1.0 mm or more, but is preferably 8.0 mm or less, more preferably 6.0 mm or less, still more preferably 4.0 mm or less. Such a solid medium can moderately stimulate the roots, and thus the advantageous effect tends to be achieved more suitably.

Herein, the average particle size of the solid medium containing a medium component refers to the average particle size by mass calculated from the particle size distribution measured in accordance with JIS Z 8815 (1994).

Non-limiting examples of the medium component in the solid medium containing a medium component include nitrogen, phosphorus, potassium, calcium, magnesium, other trace elements, vitamins, carbon sources, amino acids, and plant hormones. These may be used alone or in combinations of two or more. Preferably, the medium component(s) includes at least one selected from the group consisting of nitrogen, phosphorus, potassium, calcium, magnesium, other trace elements, vitamins, carbon sources, amino acids, and plant hormones, more preferably at least one selected from the group consisting of carbon sources, phosphorus, potassium, calcium, and amino acids, still more preferably at least one selected from the group consisting of carbon sources, phosphorus, potassium, and calcium, particularly preferably carbon sources. Here, non-limiting examples of the trace elements other than nitrogen, phosphorus, potassium, calcium, and magnesium include copper, manganese, cobalt, zinc, boron, and iron.

Non-limiting examples of the carbon sources include sugars such as sucrose, glucose, trehalose, fructose, lactose, galactose, xylose, allose, talose, gulose, altrose, mannose, idose, arabinose, apiose, and maltose. These may be used alone or in combinations of two or more. Sucrose is preferred among these.

Non-limiting examples of the vitamins include biotin, thiamine (vitamin B1), pyridoxine (vitamin B4), pyridoxal, pyridoxamine, calcium pantothenate, inositol, nicotinic acid, nicotinic acid amide, and riboflavin (vitamin B2). These may be used alone or in combinations of two or more.

Non-limiting examples of the plant hormones include those as described for the auxin plant hormones usable in the liquid medium.

The carbon source concentration in the solid medium containing a medium component is preferably at least 0.1% by mass, more preferably at least 0.5% by mass, still more preferably at least 1.0% by mass. The carbon source concentration is preferably not more than 10% by mass, more preferably not more than 8.0% by mass, still more preferably not more than 6.0% by mass. When it is within the range indicated above, the advantageous effect tends to be achieved more suitably. Herein, the term "carbon source concentration" refers to the concentration of sugars.

The plant hormone concentration in the solid medium containing a medium component is preferably not more than 2.0 mg/L, more preferably not more than 1.0 mg/L, still more preferably not more than 0.1 mg/L, particularly preferably not more than 0.08 mg/L, most preferably 0 mg/L. This can lead to a better rooting ratio.

The glutathione concentration in the solid medium containing a medium component is preferably at least 10 µmol/L, more preferably at least 30 µmol/L, still more preferably at least 40 µmol/L, particularly preferably at least 60 µmol/L, most preferably at least 80 µmol/L, but is preferably not more than 500 µmol/L, more preferably not more than 400 µmol/L, still more preferably not more than 300 µmol/L, particularly preferably not more than 200 µmol/L, most preferably not more than 150 µmol/L. This can lead to a better rooting ratio.

The solid medium containing a medium component used is preferably a medium obtained by adding a liquid medium to a solid medium as described above.

The liquid medium used may be as described for the shoot culture medium. In particular, the liquid medium is preferably a basal medium or modified basal medium, more preferably a modified basal medium, still more preferably a medium obtained by adding a carbon source to a basal medium, particularly preferably a medium obtained by adding a carbon source to MB medium, because they contain nutrients necessary for plant growth, and thus the advantageous effect can be achieved more suitably.

The liquid medium is preferably supplemented with a carbon source.

The carbon source concentration in the liquid medium is preferably at least 0.1% by mass, more preferably at least 1.0% by mass, still more preferably at least 2.0% by mass. The carbon source concentration is preferably not more than 10% by mass, more preferably not more than 7.5% by mass. When it is within the range indicated above, the advantageous effect tends to be achieved more suitably. Herein, the term "carbon source concentration" refers to the concentration of sugars.

Although the amount of the liquid medium added to the solid medium is not limited, the amount of the liquid medium per 100 parts by mass of the solid medium is preferably 10 parts by mass or more, more preferably 20 parts by mass or more, still more preferably 30 parts by mass or more, but is preferably 95 parts by mass or less, more preferably 90 parts by mass or less, still more preferably 85 parts by mass or less. When it is within the range indicated above, the advantageous effect tends to be achieved more suitably.

Preferably, the rooting induction step and the rooting induction culture step are performed, followed by further performing the rooting induction step. More preferably, the rooting induction step and the rooting induction culture step are performed, followed by further performing the rooting induction step and the rooting induction culture step in this order (in other words, these steps are performed twice). This tends to enable more suitable rooting induction of the shoots that have only been induced to form root primordia, resulting in a better shoot rooting ratio.

Preferably, the rooting induction solutions used in the multiple rooting induction steps have different compositions. This tends to lead to a better shoot rooting ratio. More specifically, preferably, the rooting induction solution used in the first rooting induction step is an auxin mixture containing indole-3-acetic acid or a solution containing no auxin hormone other than indole-3-acetic acid, and the rooting induction solution used in the second rooting induction step is a solution containing no auxin hormone other than indole-3-acetic acid or an auxin mixture containing indole-3-acetic acid. The solutions used in the first and second steps may be changed.

As described above, more suitable rooting can be achieved by performing a rooting induction step including soaking the shoots cut from the produced multiple shoot cluster in a rooting induction solution containing an auxin plant hormone and a rooting induction culture step including culturing the shoots treated in the rooting induction step in a rooting induction medium.

### EXAMPLES

The following describes preferred examples (working examples) to implement the present invention, but the scope of the present invention is not limited to the examples.

### (Examples and Comparative Examples 3 and 4)

A tissue including a shoot tip or an axillary bud is taken from a sapling of *Hevea brasiliensis.* Then, each tissue is cut into the size shown in Table 1 or 2. Next, each cut tissue is cleaned with running water and then washed with 70% by mass ethanol, followed by sterilization with an aqueous sodium hypochlorite solution diluted at approximately 5 to 10% by volume, and washing with sterile water.

Each tissue used in the experiments includes multiple buds of at least one type selected from the group consisting of apical buds, axillary buds, and dormant buds. Specifically, the tissue including a shoot tip includes an apical bud and an axillary bud, and the tissue including an axillary bud includes an axillary bud and a dormant bud.

Next, each sterilized tissue is placed in a liquid medium in a flask and subjected to shake culture (shake culture step). The liquid medium is prepared by supplementing MB basal medium (as described in Biotechnology in Agriculture and Forestry volume 5 (Trees II), pp. 222 to 245) with 5.0 mg/L of benzyladenine, 192 mg/L of glutamine, 10 mg/L of thiamine, and 3.0% by mass of sucrose and adjusting the pH of the medium to 5.7, followed by sterilizing the medium in an autoclave (121°C, 20 minutes), adding 1.0 mg/L of silver nitrate to the medium, and cooling the medium in a clean bench.

The shake culture step is performed for four weeks at a shaking speed of 120 rpm, a culture temperature of 28°C, and a light cycle of 16 hours light at 12.5 µmol/m²/s.

### (Comparative Examples 1 and 5)

A tissue having the size shown in Table 1 or 2 is used and cultured as in the examples, except that the shaking speed is 0 rpm, that is, no shaking is performed.

### (Comparative Examples 2 and 6)

A tissue having the size shown in Table 1 or 2 is used and cultured as in the examples, except that the liquid medium is replaced by a solid medium prepared by adjusting the pH of the liquid medium to 5.7, followed by adding 0.275% by mass of a gelling agent to the medium, sterilizing the medium in an autoclave (121°C, 20 minutes), and cooling the medium in a clean bench. It should be noted that the sizes of the tissues shown in the tables refer to number average values as described earlier. In the examples and comparative examples, the number average values are each calculated based on 15 tissues.

### (Shoot induction ratio)

At the fourth week of the culture period, whether or not shoots have been induced in the cultured tissues is determined, and the percentage of the cultured tissues in which shoots have been induced is calculated.

### (Multiple shoot cluster formation ratio)

At the fourth week of the culture period, the cultured tissues are evaluated to determine whether or not a multiple shoot cluster, in which multiple (e.g., two to six) shoots have been formed, has been produced from a single tissue. The percentage of the cultured tissues in which a multiple shoot cluster has been induced out of the cultured tissues in which shoots have been induced is calculated.

**[Table 1]**

| | Example 1 | Example 2 | Comparative Example 1 | Comparative Example 2 |
|---|---|---|---|---|
| Shoot culture method | Liquid medium | Liquid medium | Liquid medium | Soild medium |
| Shaking | Shaken | Shaken | Not shaken | Not shaken |
| Tissue | Tissue including shoot tip | | | |
| Size of tissue (cm) | 1.0 cm | 2.5 cm | 1.5 cm | 2.5 cm |
| Number of buds in tissue | 2 or more | 2 or more | 2 or more | 2 or more |
| Shoot induction ratio (%) | 90 | 90 | 0 | 80 |
| Multiple shoot cluster formation ratio (%) | 95 | 95 | 0 | 0 |

**[Table 2]**

| | Example 3 | Example 4 | Example 5 | Comparative Example 3 | Comparative Example 4 | Comparative Example 5 | Comparative Example 6 |
|---|---|---|---|---|---|---|---|
| Shoot culture method | Liquid medium | Liquid medium | Liquid medium | Liquid medium | Liquid medium | Liquid medium | Soild medium |
| Shaking | Shaken | Shaken | Shaken | Shaken | Shaken | Not shaken | Not shaken |
| Tissue | Tissue including axillary bud | | | | | | |
| Size of tissue (cm) | 1.5 cm | 2.5 cm | 4.5 cm | Less than 1.0 cm | 5.5 cm | 1.5 cm | 2.5 cm |
| Number of buds in tissue | 2 or more | 3 or more | 4 or more | 1 | 2 or more | 2 or more | 2 or more |
| Shoot induction ratio (%) | 50 | 70 | 70 | 50 | 50 | 0 | 80 |
| Multiple shoot cluster formation ratio (%) | 50 | 80 | 80 | 0 | 0 | 0 | 0 |

Tables 1 and 2 show that in the examples, a shake culture step is performed which includes shake-culturing a 1.0 cm to 5.0 cm tissue taken from *Hevea brasiliensis,* which has at least one bud selected from the group consisting of apical buds, axillary buds, and dormant buds, in a MB basal medium or modified medium thereof containing a cytokinin plant hormone as a liquid medium, and as a result, elongation of the multiple shoot primordia present in the tissue is induced to enable the production of a multiple shoot cluster.

### (Examples 2-1 to 2-4)

The multiple shoot cluster obtained in Example 2 or 5 is subjected to the following experiments.

In a culture vessel, a treatment solution is prepared by adding hormone(s) as shown in Table 3 to 1/2 MS medium having half the component contents of MS medium. Then, the shoots (about 15 mm in length) obtained from the multiple shoot cluster are soaked so that about half of each shoot is submerged in the solution (40% to 60% of the volume of each shoot, taken as 100%, is submerged in the rooting induction solution while it is allowed to stand still (28°C, a light cycle of 16 hours light at 12.5 µmol/m²/s)).

As for the soaking duration, which can be changed depending on the shoots, the shoots are allowed to stand still for about 20 to 72 hours.

After the treatment, the shoots are inserted into a solid medium (plant hormone concentration: 0 mg/L) prepared by supplementing MB medium with 3% sucrose and 100 µM GSH (reduced glutathione), and they are cultured for about two weeks at a culture temperature of 28°C under a cycle of 16 hours light and 8 hours dark.

After the culture, the shoots are further soaked in a treatment solution prepared by adding hormone(s) as shown in Table 3 to 1/2 MS medium, and they are allowed to stand still for 20 to 72 hours. Here, the shoots are soaked while they are allowed to stand still (28°C, a light cycle of 16 hours light at 12.5 µmol/m²/s) so that 40% to 60% of the volume of each shoot, taken as 100%, is submerged in the rooting induction solution.

After the treatment, the shoots are inserted into the surface of a solid medium containing a medium component (plant hormone concentration: 0 mg/L, wet density: 0.74 g/cm³, average particle size: 0.25 to 8.0 mm) and cultured. The solid medium is prepared by filling a culture pot with vermiculite and sterilizing it in an autoclave, followed by adding to 100 parts by mass of the vermiculite 80 parts by mass of a liquid medium prepared by supplementing MB medium with 3% by mass sucrose and 100 µM GSH (reduced glutathione). The culture is performed at a culture temperature of 28°C under a cycle of 16 hours light and 8 hours dark.

As a result of the culture, rooting can be observed after a lapse of 2 to 8 weeks, and rooted saplings can be obtained from the shoots of *Hevea brasiliensis.*

**[Table 3]**

| | Example 2-1 | Example 2-2 | Example 2-3 | Example 2-4 | Example 2-5 | Example 2-6 |
|---|---|---|---|---|---|---|
| Culture method | Soaking + Hormone free medium | Soaking + Hormone free medium | Soaking + Hormone free medium | Soaking + Hormone free medium | Soaking + Hormone free medium | Soaking + Hormone free medium |
| Hormone used (First conditions) | Indole-3-butyric acid 1-Naphthaleneacetic acid Indole-3-acetic acid | Indole-3-acetic acid | Indole-3-acetic acid | Indole-3-acetic acid Zeatin | Indole-3-butyric acid 1-Naphthaleneacetic acid Indole-3-acetic acid | Indole-3-butyric acid 1-Naphthaleneacetic acid Indole-3-acetic acid |
| Hormone used (Second conditions) | Indole-3-butyric acid 1-Naphthaleneacetic acid Indole-3-acetic acid | Indole-3-butyric acid 1-Naphthaleneacetic acid Indole-3-acetic acid | Indole-3-butyric acid 1-Naphthaleneacetic acid Indole-3-acetic acid | Indole-3-butyric acid 1-Naphthaleneacetic acid Indole-3-acetic acid | Indole-3-acetic acid | Indole-3-acetic acid |
| Hormone concentration (First) | 5 mg/l each | 15 mg/l | 20 mg/l | 15 mg/l | 5 mg/l each | 5 mg/l each |
| | | | | 0.1 mg/l | | |
| Hormone concentration (Second) | 5 mg/l each | 5 mg/l each | 5 mg/l each | 5 mg/l each | 15 mg/l | 20 mg/l |
| Rooting induction ratio | 20% | 30% | 30% | 40% | 40% | 30% |

Table 3 shows that suitable shoot rooting can be achieved by performing a rooting induction step including soaking the shoots cut from the produced multiple shoot cluster in a rooting induction solution containing an auxin plant hormone and a rooting induction culture step including culturing the shoots treated in the rooting induction step in a rooting induction medium.

Exemplary embodiments of the present invention include:
Embodiment 1. A method for producing a multiple shoot cluster, the method including
   a shake culture step including shake-culturing a 1.0 cm to 5.0 cm tissue taken from *Hevea brasiliensis,* the tissue including at least one bud selected from the group consisting of apical buds, axillary buds, and dormant buds, in a MB basal medium or modified medium thereof containing a cytokinin plant hormone as a liquid medium.
Embodiment 2. The method for producing a multiple shoot cluster according to Embodiment 1, wherein the shake-culturing is performed at a shaking speed of 50 rpm to 200 rpm.
Embodiment 3. The method for producing a multiple shoot cluster according to Embodiment 1 or 2, wherein the liquid medium contains 3.0 to 10% by mass of sucrose.
Embodiment 4. The method for producing a multiple shoot cluster according to any one of Embodiments 1 to 3, wherein the liquid medium contains at least one selected from the group consisting of amino acids and vitamins.
Embodiment 5. The method for producing a multiple shoot cluster according to any one of Embodiments 1 to 4, wherein the shake culture step is performed for at least one week.
Embodiment 6. The method for producing a multiple shoot cluster according to any one of Embodiments 1 to 5, wherein the tissue includes an apical bud.
Embodiment 7. A method for producing a clonal sapling, the method including:
   producing a multiple shoot cluster by the method for producing a multiple shoot cluster according to any one of Embodiments 1 to 6; and
   a rooting step including rooting shoots cut from the produced multiple shoot cluster.
Embodiment 8. The method for producing a clonal sapling according to Embodiment 7, wherein the rooting step includes:
   a rooting induction step including soaking the shoots cut from the produced multiple shoot cluster in a rooting induction solution containing an auxin plant hormone; and
   a rooting induction culture step including culturing the shoots treated in the rooting induction step in a rooting induction medium.
Embodiment 9. The method for producing a clonal sapling according to Embodiment 8, wherein an auxin plant hormone concentration in the rooting induction solution is 15 to 20 mg/L.
Embodiment 10. The method for producing a clonal sapling according to Embodiment 8 or 9, wherein the auxin plant hormone is indole-3-acetic acid.
Embodiment 11. The method for producing a clonal sapling according to any one of Embodiments 8 to 10, wherein the rooting induction solution contains a cytokinin plant hormone.
Embodiment 12. The method for producing a clonal sapling according to Embodiment 11, wherein the cytokinin plant hormone is zeatin.
Embodiment 13. The method for producing a clonal sapling according to any one of Embodiments 8 to 12, wherein the rooting induction step and the rooting induction culture step are performed, followed by further performing the rooting induction step.
Embodiment 14. The method for producing a clonal sapling according to Embodiment 13, wherein the rooting induction solutions used in the multiple rooting induction steps have different compositions.
Embodiment 15. The method for producing a clonal sapling according to any one of Embodiments 8 to 14, wherein in the rooting induction step, the shoots are soaked so that an end of each shoot is submerged in the rooting induction solution.
Embodiment 16. The method for producing a clonal sapling according to any one of Embodiments 8 to 15, wherein in the rooting induction step, the shoots are soaked so that 30% to 70% of the volume of each shoot, taken as 100%, is submerged in the rooting induction solution.

## Claims

1. A method for producing a multiple shoot cluster, the method comprising
a shake culture step comprising shake-culturing a 1.0 cm to 5.0 cm tissue taken from *Hevea brasiliensis,* the tissue comprising at least one bud selected from the group consisting of apical buds, axillary buds, and dormant buds, in a MB basal medium or modified medium thereof containing a cytokinin plant hormone as a liquid medium.

2. The method for producing a multiple shoot cluster according to claim 1,
wherein the shake-culturing is performed at a shaking speed of 50 rpm to 200 rpm.

3. The method for producing a multiple shoot cluster according to claim 1 or 2,
wherein the liquid medium contains 3.0 to 10% by mass of sucrose.

4. The method for producing a multiple shoot cluster according to any one of claims 1 to 3,
wherein the liquid medium contains at least one selected from the group consisting of amino acids and vitamins.

5. The method for producing a multiple shoot cluster according to any one of claims 1 to 4,
wherein the shake culture step is performed for at least one week.

6. The method for producing a multiple shoot cluster according to any one of claims 1 to 5,
wherein the tissue comprises an apical bud.

7. A method for producing a clonal sapling, the method comprising:
producing a multiple shoot cluster by the method for producing a multiple shoot cluster according to any one of claims 1 to 6; and
a rooting step comprising rooting shoots cut from the produced multiple shoot cluster.

8. The method for producing a clonal sapling according to claim 7,
wherein the rooting step comprises:
a rooting induction step comprising soaking the shoots cut from the produced multiple shoot cluster in a rooting induction solution containing an auxin plant hormone; and
a rooting induction culture step comprising culturing the shoots treated in the rooting induction step in a rooting induction medium.

9. The method for producing a clonal sapling according to claim 8,
wherein an auxin plant hormone concentration in the rooting induction solution is 15 to 20 mg/L.

10. The method for producing a clonal sapling according to claim 8 or 9,
wherein the auxin plant hormone is indole-3-acetic acid.

11. The method for producing a clonal sapling according to any one of claims 8 to 10,
wherein the rooting induction solution contains a cytokinin plant hormone.

12. The method for producing a clonal sapling according to any one of claims 8 to 11,
wherein the rooting induction step and the rooting induction culture step are performed, followed by further performing the rooting induction step.

13. The method for producing a clonal sapling according to claim 12,
wherein the rooting induction solutions used in the multiple rooting induction steps have different compositions.

14. The method for producing a clonal sapling according to any one of claims 8 to 13,
wherein in the rooting induction step, the shoots are soaked so that an end of each shoot is submerged in the rooting induction solution.

15. The method for producing a clonal sapling according to any one of claims 8 to 14,
wherein in the rooting induction step, the shoots are soaked so that 30% to 70% of the volume of each shoot, taken as 100%, is submerged in the rooting induction solution.
